# EUROPEAN PATENT SPECIFICATION

(11) **EP 2 032 183 B1**
(45) Date of publication and mention of the grant of the patent: **13.11.2013**
(21) Application number: 07795766.0
(22) Date of filing: 05.06.2007
(51) Int. Cl.: A61L 31/14, A61L 27/30, A61L 27/54, A61L 31/08, A61L 31/16, A61L 27/56

(54) **MICROPOROUS COATING ON MEDICAL DEVICES**
MIKROSPORÖSE BESCHICHTUNG AUF MEDIZINISCHEN VORRICHTUNGEN
REVÊTEMENT MICROPOREUX SUR APPAREILS MÉDICAUX

(30) Priority: 05.06.2006 US 447829
(43) Date of publication of application: 11.03.2009
(73) Proprietor: Abbott Cardiovascular Systems Inc., Santa Clara, CA 95054-2807 (US)
(72) Inventor: KLEINER, Lothar, W., Los Altos, California 94022 (US); HOSSAINY, Syed, Faiyaz, Ahmed, Fremont, California 94555 (US); ASTAFIEVA, Irina, Palo Alto, California 94306 (US); PACETTI, Stephen, D., San Jose, California 95130 (US); GLAUSER, Thierry, Redwood City, California 94062 (US); DESNOYER, Jessica, Renee, San Jose, California 95129 (US)
(74) Representative: ZBM Patents
(86) International application number: PCT/US2007/013264
(87) International publication number: WO 2007/145961

(56) References cited:
- EP-A1- 1 319 416
- WO-A-2007/130786
- US-A1- 2002 133 224
- US-A1- 2002 198 601
- US-A1- 2004 088 038
- US-A1- 2005 107 869
- US-A1- 2005 266 040

## Description

### Field of the Invention

This invention is generally related to microporous coatings on medical devices, such as drug releasing vascular stents.

### Description of the State of the Art

Stents are used not only as a mechanical intervention of vascular conditions but also as a vehicle for providing biological therapy. As a mechanical intervention, stents act as scaffoldings, functioning to physically hold open and, if desired, to expand the wall of the passageway. Typically, stents are capable of being compressed, so that they can be inserted through small vessels via catheters, and then expanded to a larger diameter once they are at the desired location. Examples in patent literature disclosing stents which have been applied in Percutaneous Transluminal Coronary Angioplasty (PTCA) procedures include stents illustrated in U.S. Patent No. 4,733,665 issued to Palmaz, U.S. Patent No. 4,800,882 issued to Gianturco, and U.S. Patent No. 4,886,062 issued to Wiktor.

US2004/088038 discloses a porous metallic intravascular stent loaded with therapeutic agents and wherein a thin layer of a polymeric material is applied to the outer surface of the tubular stent member.

US2002/198601 discloses a process for creating a microporous surface on a metallic medical device, i.e. a stent, by using an inorganic porogen salt.

US2002/133224 discloses a metallic stent encapsulated with a microporous polymeric membrane made of polyurethane and loaded with variable concentrations of one or more pharmacotherapeutic agents.

US2005/266040 discloses a medical device, i.e. a stent, comprising a metallic microporous coating loaded with therapeutic agents.

US2005/107869 discloses a stent comprising a scaffold and a substance reservoir present over said scaffold.

WO2007/130786 discloses a porous metallic intravascular stent loaded with therapeutic agents and wherein a thin layer of a polymeric material is applied to the outer surface of the tubular stent member.

Biological therapy can be achieved by medicating the stents. Medicated stents provide for the local administration of a therapeutic substance at the diseased site. In order to provide an efficacious concentration to the treated site, systemic administration of such medication often produces adverse or toxic side effects on the patient. Local delivery is a preferred method of treatment in that smaller total levels of medication are administered in comparison to systemic dosages, but are concentrated at a specific site. Local delivery thus produces fewer side effects and achieves more favorable results.

In many patients, especially diabetic patients, stentable lesions are focal manifestations of widespread vascular disease. The advent of drug delivery stents has brought relief from restenosis of the treated lesion, but leaves progression of regional vascular disease unaddressed. In addition, currently, the majority of the drug delivery systems are for hydrophobic drugs. The controlled release of hydrophilic bioactive agents is more difficult to control without an initial burst where above 40% of the agent is released in the first 24 hours for a system that should control the release over a period of 30 days or so. Some systems use block copolymers with hydrophilic and hydrophobic domains, which, theoretically, can be synthesized to help incorporate hydrophilic drugs within and then control the release of the drugs from a polymeric carrier. However, it is difficult to develop systems where both the polymer and the hydrophilic drug are compatible with coating solutions. Hydrophilic drugs attract water and can generate considerable osmotic pressure within the coating. This contributes to a burst release so the system must be robust enough to accommodate this osmotic pressure as well as mechanical strain from stent expansion. Other challenges include possible phase separation of the block-copolymer coating with hydrophilic drugs and limited mechanical integrity of such a coating.

The embodiments described below address the above-identified problems.

### SUMMARY

The present invention provides a microporous ceramic, metallic or glassy coating on a medical device that is durable or absorbable. The pores can be loaded with a agent such as a drug, which can be hydrophilic or hydrophobic. The agent can be a low molecular weight drug or a biologic (e.g., protein or peptide). In some embodiments, the microporous coating can include a topcoat, which can further control or limit the initial burst release of the drug. The topcoat can be absorbable or durable. It is to be understood that medical devices contemplated hereunder include, but are not intended to be limited to, implantable devices comprising any suitable medical substrate that can be implanted in a human or veterinary patient.

Some examples ofbioactive agents in the microporous ceramic, metallic or glassy coating can include, but are not limited to, paclitaxel, docetaxel, estradiol, nitric oxide donors, super oxide dismutases, super oxide dismutases mimics, 4-amino-2,2,6,6-tetramethylpiperidine-1-oxyl (4-amino-TEMPO), tacrolimus, dexamethasone, rapamycin, rapamycin derivatives, 40-*O*-(2-hydroxy)ethyl-rapamycin (everolimus), 40-*O*-(3-hydroxy)propyl-rapamycin, 40-*O-*[2-(2-hydroxy)ethoxy]ethyl-rapamycin, and 40-*O-*tetrazole-rapamycin, 40-epi-(N1-tetrazolyl)-rapamycin (ABT-578), pimecrolimus, imatinib mesylate, midostaurin, clobetasol, bioactive RGD or cyclic RGD, CD-34 antibody, abciximab (REOPRO), progenitor cell capturing antibody, prohealing drugs, prodrugs thereof, co-drugs thereof, or a combination thereof.

The medical device described herein can be used to treat, prevent, or ameliorate a disorder such as a vascular medical condition. Some exemplary disorders are atherosclerosis, thrombosis, restenosis, hemorrhage, vascular dissection or perforation, vascular aneurysm, vulnerable plaque, chronic total occlusion, claudication, anastomotic proliferation for vein and artificial grafts, bile duct obstruction, urethra obstruction, tumor obstruction, or combinations thereof.

### DETAILED DESCRIPTION

The present invention provides a microporous ceramic, metallic or glassy coating on a medical device that is durable or absorbable as mentioned in claim 1. The pores are loaded with an agent such as a drug, which can be hydrophilic or hydrophobic. The agent can be a low molecular weight drug or a biologic (e.g., protein or peptide). In some embodiments, the microporous coating can include a topcoat, which can further control or limit the initial burst release of the drug. The topcoat can be absorbable or durable. It is to be understood that medical devices contemplated hereunder include but are not intended to be limited to, implantable devices comprising any suitable medical substrate that can be implanted in a human or veterinary patient.

Some examples of bioactive agents in the microporous ceramic, metallic or glassy coating can include, but are not limited to, paclitaxel, docetaxel, estradiol, nitric oxide donors, super oxide dismutases, super oxide dismutases mimics, 4-amino-2,2,6,6-tetramethylpiperidine-1-oxyl (4-amino-TEMPO), tacrolimus, dexamethasone, rapamycin, rapamycin derivatives, 40-*O*-(2-hydroxy)ethyl-rapamycin (everolimus), 40-*O*-(3-hydroxy)propyl-rapamycin, 40-*O*-[2-(2-hydroxy)ethoxy]ethyl-rapamycin, and 40-*O-*tetrazole-rapamycin, 40-epi-(N1-tetrazolyl)-rapamycin (ABT-578), pimecrolimus, imatinib mesylate, midostaurin, clobetasol, bioactive RGD or cyclic RGD, CD-34 antibody, abciximab (REOPRO), progenitor cell capturing antibody, prohealing drugs, prodrugs thereof, co-drugs thereof, or a combination thereof.

The medical device described herein can be used to treat, prevent, or ameliorate a disorder such as a vascular medical condition. Some exemplary disorders are atherosclerosis, thrombosis, restenosis, hemorrhage, vascular dissection or perforation, vascular aneurysm, vulnerable plaque, chronic total occlusion, claudication, anastomotic proliferation for vein and artificial grafts, bile duct obstruction, urethra obstruction, tumor obstruction, or combinations thereof.

As used herein, the term "ceramic" or "glassy" includes a coating formed from an inorganic matrix or a matrix that includes an inorganic material or combinations of inorganic materials. Such an inorganic material can be any biocompatible inorganic material, which can be hydroxyapatite, dahlite, brushite, octacalcium phosphate, tricalcium phosphate, calcium sulphate, bioglasses, carbides, tungsten carbide, carbon, porous matrices of biodegradable metals comprised primarily of iron, zinc, magnesium, or alloys thereof. Bioglasses that can be included in the coating include, e.g., Bioglass 45S5, Bioglass 45S5F, Bioglass 45S5.4F, Bioglass 40S5B5, Bioglass 52S4.6, Bioglass 55S4.3, Ceravital KGC, Ceravital KGS, Ceravital Kgy213, A-W Glass Ceramic, MB Glass Ceramic, Bioglasses, and compositions of SiO₂/NaO₂/CaO/P₂O₅.

The term "microporous" refers to an attribute of coating having pores in a size ranging from 20 nm to above 500 µm, for example, from 50 nm to 1 µm or from above 1 µm to 100 µm.

In some embodiments, the glassy or ceramic coating can include a metal or metal fiber such as iron (Fe), magnesium (Mg), aluminum (Al), zinc (Zn), calcium (Ca), manganese (Mn), titanium (Ti), zirconium (Zr), stainless steel, gold (Au), platinum (Pt), iridium (Ir), niobium (Nb), silver (Ag), tantalum (Tl), other vascular compatible metals, or combinations of these.

### Methods of forming microporous ceramic, metallic or glassy coating

The microporous ceramic, metallic or glassy coating can be formed using a variety of established methods or processes. Such processes include plasma spray, sol-gel processes, sputtering, solid-state sintering, liquid-phase sintering, chemical vapor deposition, electrochemical, electrophoresis, precipitation and dissolution of particles within the coating such as water soluble salts..

In some embodiments, the method of forming the ceramic, metallic or glassy coating can be formed by precipitating a ceramic or glassy material onto a medical device (e.g., stent), forming a layer of the ceramic, metallic or glassy coating on the medical device. For example, such precipitation can be carried out by placing a medical device in a solution including a salt, an acid or a base forming a ceramic or glassy material and adding an agent to the solution so as to form the ceramic or glassy material. The ceramic or glassy material thus formed can form a layer on the medical device.

In some embodiments, the method of forming the ceramic, metallic or glassy coating can be deposition. In the plasma spray process, a ceramic powder is suspended in a carrier gas stream. This stream is fed between two electrodes. An electric arc is formed between the two electrodes by application of a high voltage. Such a deposition process is described in, e.g., Wolke, J.G.C., et al., J. Thermal Spray Technology 1:75-82 (1992).

In some embodiments, the method of forming the ceramic coating or glassy coating can be carried out by synthesis. For example, the synthesis can be achieved via glass spinodal decomposition to produce a phase separated ceramic where one of the components can either be etched out or pyrolyzed. In this process, SiO₂, H₃BO₃ and NaCO₃ are typical ingredients used to make Na₂O-B₂O₂-SiO₂-based borosilicate glass. The homogeneous formed part or coating undergoes a spinodal decomposition when thermally treated to yield a Na₂O-B₂O₂ rich phase and a SiO₂ rich phase. The non-silica phase can be etched out using hot water or an acid solution to yield a silica rich porous part or coating.

The porosity can be created by a variety of techniques, which can be, for example, sintering. For example, in some embodiments, a slurry of magnesium particles is applied as a coating with a binder of a polymer (resin) such as polyvinyl alcohol (PVA). After application and drying, the coating can be heated to near the melting point of magnesium to sinter the particles together and burn off the binder, leaving voids between the particles. In some embodiments, the pores can be formed by use of a porogen phase in the coating process. A porogen is an inert material, often added in a particulate form, which is removed after the coating is made, rendering it porous. For example, the porogen can be included before or during the synthesis, deposition, precipitation, or sintering of the ceramic or glassy material. For example, as part of a sol-gel process, materials such as triethyl phosphite and calcium nitrate can be combined in an alcohol/water solvent with nanoparticles of a polymer (resin) (such as PLLA) and the phosphate allowed to partially hydrolyze. This solution can then be aerosolized and coated on a stent. After drying to remove the solvent, the coating can be fired (e.g., at 500 °C) which sinters the calcium phosphate ceramic and pyrolyzes the resin porogen, leaving behind pores.

The release of the agent or drug loaded in the microporous ceramic, metallic or glassy coating can be controlled by controlling the size and distribution of the pores. In some embodiments, the pores can have a size gradient. Such a size gradient can result in the pore size becoming smaller with increasing depth in the coating, or vice versa.

In some embodiments, the release of the agent can be controlled by controlling the volume fraction of coating that is pores. For example, a coating having a larger volume fraction of pores can have a faster rate of release of the agent or drug, or vice versa. In some embodiments, the microporous ceramic, metallic or glassy coating can have volume fraction of pores in the range between 0.01 and 0.5, for example, 0.05, 0.1, 0.2, 0.3, and 0.4. In some embodiments, the volume fraction of pores in the coating can be beyond 0.5.

In some embodiments, the release of the agent or drug loaded on the coating can be controlled by varying the thickness of the porous coating. Generally, the thicker the coating, the slower the release of the agent from the coating. The thickness of a porous coating also affects the load capacity of the agent in that a thicker porous coating can have a larger drug load. In some embodiments, the thickness of the ceramic, metallic or glassy coating can range from 0.1 µm to 100 µm, for example 0.2 µm to 20 µm.

The release of a drug can also be affected or controlled by controlling the tortuosity of the pores. For example, pores can be formed in different geometry or shape so as to have different release properties for an agent loaded therein.

In some embodiments, the microporous coating can have different fraction of macro pores versus micropores. Such fractions of macro pores versus micropores (macro/micro) can range from 0.01 to 0.99, e.g., 0.1, 0.2, 0.3, 0.4, 0.5, 0,6, 0.7, or 0.8. As used herein, macro pores refer to pores having a size above 1 µm, and micropores refer to pores having a size ranging from 20 nm to 1 µm. Coatings with a higher fraction of macro pores can have a higher rate of release of an agent loaded therein, and vice versa.

In some embodiments, the release of an agent loaded within the pores of the coating described herein can be controlled by varying the adsorption or chemisorption potential of the agent on the surface both inside and outside the pores. Surfaces of pores in a ceramic, metallic or glassy coating are generally polar or ionic and hydrophilic in nature and can have hydroxyl groups. Therefore, agents with polar group(s) and/or ionic group(s) can have a higher potential of adsorption to the surface of pores, leading to a slower release of the agent from the ceramic, metallic or glassy coating. One of ordinary skill in the art can determine the relative release rate of an agent loaded within the pores according to the chemical and physical nature of the agent.

In some embodiments, the microporous ceramic, metallic or glassy coating can have pores with different surface roughness factor within the pores to control the release of an agent loaded therein. Relatively speaking, pores having a rougher surface can allow a slower release of the agent loaded therein whereas pores having a more smooth surface can allow a faster release of the agent.

According to the invention, the release of an agent in the pores is controlled by a loading adsorption or chemisorption nidus within the pore structures of the microporous ceramic, metallic or glassy coating. Pores in the microporous coating described herein are loaded with a adsorption or chemisorption nidus for an agent so as to control (to lower) the release of the agent from the coating. As used herein, the term "nidi" or "nidus" refers to a chemical or material to which an agent loaded within the pores is adsorbed. Such nidus chemicals can be, for example, fullerene or activated carbon, zeolites, alumino silicates, clays, chromium (Cr), silica, alumina, titania, gold (Au) or manganese (Mn), calcium carbonate or combinations of these.

In some embodiments, release of an agent loaded within pores in a coating described herein can be controlled by using a rate limiting thin polymer topcoat. Such a topcoat can comprise a polymer which can be degradable or biodurable. In some embodiments, the topcoat can include 25 µg to 200 µg polymer. Exemplary polymers for forming the topcoat can be poly(D,L-lactic acid), poly(D,L-lactide), poly(L-lactide), poly(D,L-lactide-co-glycolide), poly(L-lactide-co-glycolide), poly(glycolide), poly(caprolactone), poly(ester amide), poly(vinylidene fluoride-co-hexafluoropropylene) (PVPF-HFP), poly(butyl methacrylate) (PBMA), or combinations thereof. Other polymers useful for forming the topcoat are described below. In some embodiments, the topcoat can include a biobeneficial material. A biobeneficial material is one which enhances the biocompatibility of the particles or device by being non-fouling, hemocompatible, actively non-thrombogenic, or antiinflammatory, all without depending on the release of a pharmaceutically active agent.

In some embodiments, the agent to be loaded within the pores in the coating described herein can be included in a microparticulate or nanoparticulate polymer matrix or encapsulated within microcapsulates formed from polymers. Such polymers can be degradable or biodurable. In some embodiments, the polymers can be, for example, poly(D,L-lactic acid), poly(D,L-lactide), poly(L-lactide), poly(D,L-lactide-co-glycolide), poly(L-lactide-co-glycolide), poly(glycolide), poly(caprolactone), poly(ester amide), poly(vinylidene fluoride-co-hexafluoropropylene) (PVDF-HFP), poly(butyl methacrylate) (PBMA), or combinations thereof. Other polymers useful for forming the topcoat are described below. The polymer matrix or polymer microcapsules can be formed in a size that can be loaded within the pores of the microporous coating described herein. Release of the agent can then be controlled by at least two factors, namely, (1) release rate of the particulate polymer matrix or capsules from the microporous coating, and (2) release rate of the agent from the polymer matrix or capsules. A topcoat over the microporous coating is optional, but can provide further release rate control of the hydrophobic and/or hydrophilic agents.

In some embodiments, release of an agent included in the microporous ceramic, metallic or glassy coating described herein can be additionally controlled by controlling the absorption rate of ceramic, metallic or glassy coating. The absorption rate of the coating can be controlled by a variety of factors. For example, the chemical composition of the coating can be varied or tuned by incorporating an amount of a metallic material. Such metallic materials can be, for example, iron (Fe), magnesium (Mg), aluminum (Al), zinc (Zn), calcium (Ca), manganese (Mn), titanium (Ti), zirconium (Zr), stainless steel, gold (Au), platinum (Pt), iridium (Ir), niobium (Nb), silver (Ag), tantalum (Tl), other vascular compatible metals, or combinations in general. In some embodiments, the coating composition can include a biodegradable polymeric material. Such a polymeric material can be, for example, poly(D,L-lactic acid), poly(D,L-lactide), poly(L-lactide), poly(D,L-lactide-co-glycolide), poly(L-lactide-co-glycolide), poly(glycolide), poly(caprolactone), poly(ester amide), or other degradable polymers described below. In some embodiments, the absorption of the microporous ceramic, metallic or glassy coating can be controlled by a coating sequence that includes polymer and ceramic and/or glassy coatings in a layer-by-layer sequence. The polymer can be degradable or durable. The degradation of polymer can facilitate or speed up the absorption of the ceramic, metallic or glassy coating. When the polymer is durable, a layer of polymer can inhibit or slow down the absorption of the ceramic, metallic or glassy coating.

In some embodiments, the release rate of an agent loaded within the pores of the coating described herein can be additionally controlled by process parameters in forming the coating. Such parameters include, e.g., temperature, pressure, humidity, or solvent environment.

### Loading of agents

The agent can be loaded within the pores of the ceramic, metallic or glassy coating described herein by a variety of established methods or procedures. For example, the agent can be loaded after the porogen phase is removed in the process of forming the ceramic, metallic or glassy coating. Such a porogen phase, which was described above, can be used in the synthesis of the ceramic or glassy material, deposition of the ceramic or glassy material onto the surface of a medical device, precipitation of the ceramic or glassy material onto the surface of a medical device, or sintering of the ceramic or glassy microporous coating.

In some embodiments, the agent can be loaded in the pores by assistance of physical means. For example, a drug solution, which can be a saturated solution or unsaturated solution, can be forced into the pores of the ceramic, metallic or glassy coating either by using pressure or by first conditioning the stent under vacuum (vacuum infiltration). Once the ceramic is loaded, the solvent can be removed by, e.g., evaporation, leaving the drug within the pores of the microporous ceramic, metallic or glassy coating. The pressure can be in the range between 30 psi to 2000 psi. The pressure can have a broad range and depends upon the pore size, the interfacial properties of the pore surface and solution as well as the viscosity of the solution with contains the active.

In some embodiments, where the agent has the requisite thermal stability, the coating can be exposed to a molten solution of a neat agent (e.g., drug) and a vacuum can then be applied to the system. After the drug infiltrated the pores of the ceramic or glassy coating, the vacuum can be released with an inert gas. In some embodiments, the molten drug can be kept in an inert gas to keep stable. The porous ceramic coating is dipped into the molten agent to load the pores with drug. The loading may of may not require pressure to ensure that the loading is complete. In some embodiments, the loading described in these embodiments can be achieved with a heated solution of an agent with nominal solvent. As used herein, nominal solvent refers to a content of solvent in the range between above 0% and 10%.

In some embodiments, the pores in the ceramic, metallic or glassy coating can have an ion exchange property. If the agent is ionic, the agent can be loaded within the pores by an ion exchange process. For example, a drug solution can be exposed to a porous coating. The drug can then be ionically loaded within the pores either by a more favorable ionic interaction or by selectively removing the counterion(s) initially present on the surface of the pores in the ceramic, metallic or glassy coating. The ion exchange process is well established in the art. An ordinary artisan can readily carry out the ion exchange to load an ionic agent into the pores in the ceramic, metallic or glassy coating.

In some further embodiments, an agent can be allowed to diffuse into the pores in a ceramic, metallic or glassy coating. The agent can then be bound to the matrix of the pores in a ceramic, metallic or glassy coating by a force such as hydrogen bonding, Van-der-Waals interaction, or affinity interaction such as like-like interaction. The pores can be infiltrated by a solution of the active agent by ay of the techniques previously described. Then, the active agent can precipitated inside the pores by a change in pH, change in temperature, change in ionic strength, or addition of specific agents which cause the active agent to precipitate.

### Bioactive Agents

The agent that can be loaded into the pores of the microporous ceramic, metallic or glassy coating described herein can be therapeutic, prophylactic, or diagnostic agent(s). These agents can have anti-proliferative or anti-inflammatory properties or can have other properties such as antineoplastic, antiplatelet, anti-coagulant, anti-fibrin, antithrombogenic, antimitotic, antibiotic, antiallergic, antifibrotic, and antioxidant. The agents can be cystostatic agents, agents that promote the healing of the endothelium such as NO releasing or generating agents, agents that attract endothelial progenitor cells, agents that promote the attachment, migration or proliferation of endothelial cells (e.g., natriuretic peptides such as CNP, ANP or BNP peptide or an RGD or cRGD peptide), while impeding smooth muscle cell proliferation. Examples of suitable therapeutic and prophylactic agents include synthetic inorganic and organic compounds, proteins and peptides, polysaccharides and other sugars, lipids, and DNA and RNA nucleic acid sequences having therapeutic, prophylactic or diagnostic activities. Some other examples of the bioactive agent include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides, small interfering RNA (siRNA), small hairpin RNA (shRNA), aptamers, ribozymes and retroviral vectors for use in gene therapy. Examples of anti-proliferative agents include rapamycin and its functional or structural derivatives, 40-*O*-(2-hydroxy)ethyl-rapamycin (everolimus), and its functional or structural derivatives, paclitaxel and its functional and structural derivatives. Examples of rapamycin derivatives include 40-epi-(N1-tetrazolyl)-rapamycin (ABT-578), 40-*O*-(3-hydroxy)propyl-rapamycin, 40-*O*-[2-(2-hydroxy)ethoxy]ethyl-rapamycin, and 40-*O-*tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin^{®} from Pharmacia & Upjohn, Peapack N.J.), and mitomycin (e.g. Mutamycin^{®} from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as Angiomax (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine, (a PDGF antagonist), nitric oxide or nitric oxide donors, super oxide dismutases, super oxide dismutase mimetic, 4-amino-2,2,6,6-tetramethylpiperidine-1-oxyl (4-amino-TEMPO), estradiol, anticancer agents, dietary supplements such as various vitamins, and a combination thereof. Examples of anti-inflammatory agents including steroidal and non-steroidal anti-inflammatory agents include tacrolimus, dexamethasone, clobetasol, mometasone, or combinations thereof. Examples of cytostatic substances include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Prinivil^{®} and Prinzide^{®} from Merck & Co., Inc., Whitehouse Station, NJ). An example of an antiallergic agent is permirolast potassium. Other therapeutic substances or agents which may be appropriate include alpha-interferon, pimecrolimus, imatinib mesylate, midostaurin, bioactive RGD, SIKVAV peptides, elevating agents such as cANP or cGMP peptides, and genetically engineered endothelial cells. The foregoing substances can also be used in the form of prodrugs or co-drugs thereof. The foregoing substances also include metabolites thereof and/or prodrugs of the metabolites. The foregoing substances are listed by way of example and are not meant to be limiting. Other active agents which are currently available or that may be developed in the future are equally applicable.

The dosage or concentration of the bioactive agent required to produce a favorable therapeutic effect should be less than the level at which the bioactive agent produces toxic effects and greater than non-therapeutic levels. The dosage or concentration of the bioactive agent can depend upon factors such as the particular circumstances of the patient, the nature of the trauma, the nature of the therapy desired, the time over which the administered ingredient resides at the vascular site, and if other active agents are employed, the nature and type of the substance or combination of substances. Therapeutically effective dosages can be determined empirically, for example by infusing vessels from suitable animal model systems and using immunohistochemical, fluorescent or electron microscopy methods to detect the agent and its effects, or by conducting suitable in vitro studies. Standard pharmacological test procedures to determine dosages are understood by one of ordinary skill in the art.

### Biocompatible polymers

The biocompatible polymer that can be used herein can be biodegradable (either bioerodable or bioabsorbable or both) or nondegradable and can be hydrophilic or hydrophobic. Representative biocompatible polymers include, but are not limited to, poly(ester amide), polyhydroxyalkanoates (PHA), poly(3-hydroxyalkanoates) such as poly(3-hydroxypropanoate), poly(3-hydroxybutyrate), poly(3-hydroxyvalerate), poly(3-hydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(D,L-lactide), poly(L-lactide), polyglycolide, poly(D,L-lactide-co-glycolide), poly(L-lactide-co-glycolide), polycaprolactone, poly(lactide-co-caprolactone), poly(glycolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), polycyanoacrylates, poly(trimethylene carbonate), poly(iminocarbonate), polyphosphazenes, silicones, polyesters, polyolefins, polyisobutylene and ethylene-alphaolefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, poly(glyceryl sebacate), poly(propylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), epoxy resins, polyurethanes, rayon, rayon-triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. poly(ethylene oxide-co-lactic acid) (PEO/PLA)), polyalkylene oxides such as poly(ethylene oxide), poly(propylene oxide), poly(ether ester), polyalkylene oxalates, phosphoryl choline containing polymer, choline, poly(aspirin), polymers and co-polymers of hydroxyl bearing monomers such as 2-hydroxyethyl methacrylate (HEMA), hydroxypropyl methacrylate (HPMA), hydroxypropylinethacrylamide, PEG acrylate (PEGA), PEG methacrylate, methacrylate polymers containing 2-methacryloyloxyethylphosphorylcholine (MPC) and *n*-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC™ surfactants (polypropylene oxide-co-polyethylene glycol), poly(tetramethylene glycol), hydroxy functional poly(vinyl pyrrolidone), molecules such as collagen, chitosan, alginate, fibrin, fibrinogen, cellulose, starch, dextran, dextrin, hyaluronic acid, fragments and derivatives of hyaluronic acid, heparin, fragments and derivatives of heparin, glycosamino glycan (GAG), GAG derivatives, polysaccharide, elastin, elastin protein mimetics, or combinations thereof. Some examples of elastin protein mimetics include (LGGVG)ₙ, (VPGVG)ₙ, Val-Pro-Gly-Val-Gly, or synthetic biomimetic poly(L-glytanmate)-b-poly(2-acryloyloxyethyllactoside)-b-poly(1-glutamate) triblock copolymer.

In some embodiments, the polymer can be poly(ethylene-co-vinyl alcohol), poly(methoxyethyl acrylate), poly(methoxyethyl methacrylate), poly(dihydroxylpropyl methacrylate), polymethacrylamide, aliphatic polyurethane, aromatic polyurethane, nitrocellulose, poly(ester amide benzyl), co-poly-{[N,N'-sebacoyl-bis-(L-leucine)-1,6-hexylene diester]_{0.75}-[N,N'-sebacoyl-L-lysine benzyl este]_{0.25}} (PEA-Bz), co-poly-{[N,N'-sebacoyl-bis-(L-leucine)-1,6-hexylene diester]_{0.75}-[N,N'-sebacoyl-L-lysine-4-amino-TEMPO amide]_{0.25}} (PEA-TEMPO), aliphatic polyester, aromatic polyester, fluorinated polymers such as poly(vinylidene fluoride-co-hexafluoropropylene), poly(vinylidene fluoride) (PVDF), and Teflon™ (polytetrafluoroethylene), a biopolymer such as elastin mimetic protein polymer, star or hyper-branched SIBS (styrene-block-isobutylene-block-styrene), or combinations thereof. In some embodiments, where the polymer is a copolymer, it can be a block copolymer that can be, e.g., di-, tri-, tetra-, or oligo block copolymers or a random copolymer. In some embodiments, the polymer can also be branched polymers such as star polymers.

In some embodiments, a coating having the features described herein can exclude any one of the aforementioned polymers.

As used herein, the terms poly(D,L-lactide), poly(L-lactide), poly(D,L-lactide-co-glycolide), and poly(L-lactide-co-glycolide) can be used interchangeably with the terms poly(D,L-lactic acid), poly(L-lactic acid), poly(D,L-lactic acid-co-glycolic acid), or poly(L-lactic acid-co-glycolic acid), respectively.

### Biobeneficial Material

The biobeneficial material that can be used in the present invention can be a polymeric material or non-polymeric material. The biobeneficial material is preferably non-toxic, non-antigenic and non-immunogenic.

Representative biobeneficial materials include, but are not limited to, polyethers such as poly(ethylene glycol), copoly(ether-esters) (e.g. PEO/PLA), polyalkylene oxides such as poly(ethylene oxide), poly(propylene oxide), poly(ether ester), polyalkylene oxalates, polyphosphazenes, phosphoryl choline, choline, poly(aspirin), polymers and copolymers of hydroxyl bearing monomers such as hydroxyethyl methacrylate (HEMA), hydroxypropyl methacrylate (HPMA), hydroxypropylmethacrylamide, poly(ethylene glycol)acrylate (PEGA), PEG methacrylate, 2-methacryloyloxyethylphosphorylcholine (MPC) and n-vinyl pyrrolidone (VP), methacrylate copolymers with MPC, copolymers containing methacryloyl sulfobetaine, carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC™ surfactants (polypropylene oxide-co-polyethylene glycol), poly(tetramethylene glycol), hydroxy functional poly(vinyl pyrrolidone), molecules such as fibrin, fibrinogen, cellulose, starch, collagen, dextran, dextrin, hyaluronic acid, fragments and derivatives of hyaluronic acid, heparin, fragments and derivatives of heparin, glycosamino glycan (GAG), GAG derivatives, polysaccharide, elastin, chitosan, alginate, silicones, PolyActive™, and combinations thereof. In some embodiments, a coating described herein can exclude any one of the aforementioned polymers. The term PolyActive™ refers to a block copolymer having flexible poly(ethylene glycol) and poly(butylene terephthalate) blocks (PEGT/PBT). PolyActive™ is intended to include AB, ABA, BAB copolymers having such segments of PEG and PBT (e.g., poly(ethylene glycol)-block-poly(butyleneterephthalate)-block poly(ethylene glycol) (PEG-PBT-PEG).

In a preferred embodiment, the biobeneficial material can be a polyether such as poly(ethylene glycol) (PEG) or polyalkylene oxide.

### Examples of Implantable Device

As used herein, an implantable device may be any suitable medical substrate that can be implanted in a human or veterinary patient. Examples of such implantable devices include self-expandable stents, balloon-expandable stents, stent-grafts, grafts (e.g., aortic grafts), heart valve prostheses, cerebrospinal fluid shunts, pacemaker electrodes, catheters, and endocardial leads (e.g., FINELINE and ENDOTAK, available from Guidant Corporation, Santa Clara, CA), anastomotic devices and connectors, orthopedic implants such as screws, spinal implants, electro-stimulatory devices. The underlying structure of the device can be of virtually any design. The device can be made of a metallic material or an alloy such as, but not limited to, cobalt chromium alloy (ELGILOY), stainless steel (316L), high nitrogen stainless steel, e.g., BIODUR 108, cobalt chrome alloy L-605, "MP35N," "NIP20N," ELASTINITE (Nitinol), tantalum, nickel-titanium alloy, platinum-iridium alloy, gold, magnesium, or combinations thereof. "MP35N" and "MP20N" are trade names for alloys of cobalt, nickel, chromium and molybdenum available from Standard Press Steel Co., Jenkintown, PA. "MP35N" consists of 35% cobalt, 35% nickel, 20% chromium, and 10% molybdenum. "MP20N" consists of 50% cobalt, 20% nickel, 20% chromium, and 10% molybdenum. Devices made from bioabsorbable or biostable polymers could also be used with the embodiments of the present invention.

### Method of Use

In accordance with embodiments of the invention, the agent can be released from a medical device (e.g., stent) during delivery and (in the case of a stent) expansion of the device, or thereafter, and released at a desired rate and for a predetermined duration of time at the site of implantation.

Preferably, the medical device is a stent. The stent described herein is useful for a variety of medical procedures, including, by way of example, treatment of obstructions caused by tumors in bile ducts, esophagus, trachea/bronchi and other biological passageways. A stent having the above-described coating is particularly useful for treating diseased regions of the vascular system caused by lipid deposition, monocyte or macrophage infiltration, or dysfunctional endothelium or a combination thereof, or occluded regions of blood vessels caused by abnormal or inappropriate migration and proliferation of smooth muscle cells, thrombosis, and restenosis. Stents may be placed in a wide array of blood vessels, both arteries and veins. Representative examples of sites include the iliac, renal, carotid and coronary arteries.

For implantation of a stent, an angiogram is first performed to determine the appropriate positioning for stent therapy. An angiogram is typically accomplished by injecting a radiopaque contrasting agent through a catheter inserted into an artery or vein as an x-ray is taken. A guidewire is then advanced through the lesion or proposed site of treatment. Over the guidewire is passed a delivery catheter which allows a stent in its collapsed configuration to be inserted into the passageway. The delivery catheter is inserted either percutaneously or by surgery into the femoral artery, brachial artery, femoral vein, or brachial vein, and advanced into the appropriate blood vessel by steering the catheter through the vascular system under fluoroscopic guidance. A stent having the above-described features may then be expanded at the desired area of treatment. A post-insertion angiogram may also be utilized to confirm appropriate positioning.

## Claims

1. A medical device comprising a microporous ceramic, metallic or glassy coating,
the coating comprising micropores loaded with a bioactive agent and providing for a controlled release profile of the agent;
wherein the pores include an adsorption or chemisorption nidus selected from the group consisting of fullerenes, activated carbon, zeolite, aluminosilicate, calcium carbonate, chromium (Cr), silica, alumina, titania, gold (Au), manganese (Mn), and combinations thereof.

2. The medical devide of claim 1, wherein the coating is a ceramic or glassy coating and comprises an inorganic matrix selected from the group consisting of hydroxyapatite, dahlite, brushite, octacalcium phosphate, tricalcium phosphate, calcium sulfate, alumina, bioglasses, carbides, tungsten carbide, carbon, or combinations thereof.

3. The medical device of claim 1, wherein the coating is a metallic coating and is of a biodegradable metal comprised primarily of zinc, magnesium, or alloys thereof.

4. The medical device of claim 1, wherein the release profile is controlled by a factor selected from size distribution of the micropores, size gradient of the micropores, thickness of the coating, tortuosity of a microporous network in the coating, surface roughness factor of the micropores, adsorption or chemisorption potential of the agent onto the surface inside of the micropores, a topcoat, or a combination of these.

5. The medical device of claim 1, wherein the ceramic, metallic, or glassy coating has a volume fraction of pores ranging from 0.01 to 0.5.

6. The medical device of claim 1, wherein the ceramic, metallic or glassy coating comprises macro pores and micropores in a fraction of macro pores to micropores of 0.01 to 0.99.

7. The medical device of claim 1, further comprising a polymer topcoat over the ceramic, metallic or glassy coating.

8. The medical device of claim 1, wherein the bioactive agent is contained in a particulate polymer matrix or microcapsule.

9. The medical device of claim 1, wherein the ceramic, metallic or glassy coating further comprises a metallic material selected from the group consisting of iron (Fe), magnesium (Mg), aluminum (Al), zinc (Zn), calcium (Ca), manganese (Mn), titanium (Ti), zirconium (Zr), stainless steel, gold (Au), platinum (Pt), iridium (Ir), niobium (Nb), silver (Ag), tantalum (T1), other vascular compatible metals and combinations of these.

10. The medical device of claim 1, wherein the bioactive agent is selected from the group consisting ofpaclitaxel, docetaxel, estradiol, nitric oxide donors, super oxide dismutases, 4-amino-2,2,6,6-tetramethylpiperidine-1-oxyl (4-amino-TEMPO), tacrolimus, dexamethasone, rapamycin, 40-0-(2-hydroxy)ethyl-rapamycin (everolimus), 40-0-(3-hydroxy)propyl-rapamycin, 40-042-(2-hydroxy)ethoxy]ethyl-rapamycin, and 40-O-tetrazole-rapamycin, 40-epi-(N1-tetrazolyl)-rapamycin (ABT-578), clobetasol, pimecrolimus, imatinib mesylate, midostaurin, and combinations thereof.

11. The medical device of claim 1, which is a stent.

12. The medical device of claim 1, wherein the microporous coating is a ceramic or glassy coating comprising an inorganic matrix selected from the group consisting of hydroxyapatite, dahlite, brushite, octacalcium phosphate, tricalcium phosphate, calcium sulfate, bioglasses, carbides, tungsten carbide, carbon, and combinations thereof.

13. The medical device of claim 1, wherein the microporous ceramic, metallic or glassy coating has a gradient in the size of the micropores.

14. The medical device of claim 1, wherein the adsorption or chemisorption nidus is selected from the group consisting of activated carbon, and calcium carbonate.

15. The medical device of claim 1, wherein the adsorption or chemisorption nidus is selected from the group consisting of fullerene, and manganese.

16. The medical device of claim 1, further comprising one or more layer(s) of coating of a polymer wherein the layer(s) ofpolymer coating and the microporous ceramic, metallic or glassy coating comprises a layer-by-layer construct.

17. The medical device of claim 1, wherein the ceramic or glassy coating comprises an inorganic matrix selected from the group consisting of carbides, tungsten carbide, carbon, Bioglass 45S5, Bioglass 45S5F, Bioglass 45S5.4F, Bioglass 40S5B5, Bioglass 52S4.6, Bioglass 55S4.3, A-W Glass Ceramic, MB Glass Ceramic, compositions of SiO₂/NaO/CaO/P₂O₂, and combinations thereof.

18. The medical device of claim 1, wherein the ceramic or glassy coating comprises a metallic material selected from the group consisting zinc (Zn), manganese (Mn), stainless steel, iridium (Ir), and combinations of these.

19. A method of forming a medical device comprising:
forming a microporous ceramic, metallic or glassy coating comprising macro pores and/or micropores,
wherein the pores include an adsorption or chemisorption nidus selected from the group consisting of fullerenes, activated carbon, zeolite, aluminosilicate, calcium carbonate, chromium (Cr), silica, alumina, titania, gold (Au), manganese (Mn), and combinations thereof
and
loading a bioactive agent into the pores.

20. The method of claim 19, wherein the coating is a metallic coating and is of a biodegradable metal comprised primarily of zinc, magnesium, or alloys thereof.

21. The method of claim 19, wherein the loading comprises loading the bioactive agent into the pores by an ion exchange process.

## Patentansprüche

1. Medizinische Vorrichtung umfassend eine mikroporöse, metallische oder glasartige Beschichtung,
wobei die Beschichtung Mikroporen umfasst, die mit einem Wirkstoff geladen sind und für ein Profil kontrollierter Freisetzung des Wirkstoffes sorgen;
wobei die Poren einen Adsorptions- oder Chemisorptionsnidus umfassen, der ausgewählt ist aus der Gruppe bestehend aus Fullerenen, aktivem Kohlenstoff, Zeolit, Aliminiumsilicat, Calciumcarbonat, Chromium (Cr), Silica, Aluminiumoxid, Titandioxid, Gold (Au), Mangan (Mn) und Kombinationen davon.

2. Medizinische Vorrichtung nach Anspruch 1, wobei die Beschichtung eine keramische oder glasartige Beschichtung ist und eine anorganische Matrix umfasst, die ausgewählt ist aus der Gruppe bestehend aus Hydroxyapatit, Dahllit, Brushit, Octacalciumphosphat, Tricalciumphosphat, Calciumsulfat, Aluminiumoxid, Biogläsern, Carbiden, Tungstencarbid, Kohlenstoff oder Kombinationen davon.

3. Medizinische Vorrichtung nach Anspruch 1, wobei die Beschichtung eine metallische Beschichtung ist und aus einem biologisch abbaubaren Metall ist, das hauptsächlich aus Zink, Magnesium oder Legierungen daraus besteht.

4. Medizinische Vorrichtung nach Anspruch 1, wobei das Freisetzungsprofil mittels eines Faktors kontrolliert wird, der ausgewählt ist aus Größenverteilung der Mikroporen, Größengefälle der Mikroporen, Stärke der Beschichtung, Tortuosität des mikroporösen Netzwerks innerhalb der Beschichtung, Rauheitsfaktor der Oberfläche der Mikroporen, Absorptions- oder Chemisorptionspotential des Wirkstoffes auf die Oberfläche in den Mikroporen, einer Deckbeschichtung oder einer Kombination davon.

5. Medizinische Vorrichtung nach Anspruch 1, wobei die keramische, metallische oder glasartige Beschichtung einen Volumenanteil von Poren hat, der von 0,01 bis 0,5 reicht.

6. Medizinische Vorrichtung nach Anspruch 1, wobei die keramische, metallische oder glasartige Beschichtung Makroporen und Mikroporen mit einem Verhältnis von Makroporen zu Mikroporen von 0,01 bis 0,99 umfasst.

7. Medizinische Vorrichtung nach Anspruch 1 weiterhin umfassend eine Polymerdeckbeschichtung auf der keramischen, metallischen oder glasartigen Beschichtung.

8. Medizinische Vorrichtung nach Anspruch 1, wobei der Wirkstoff innerhalb einer partikelförmigen Polymermatrix oder Mikrokapsel enthalten ist.

9. Medizinische Vorrichtung nach Anspruch 1, wobei die keramische, metallische oder glasartige Beschichtung weiterhin einen metallischen Stoff umfasst, der ausgewählt ist aus der Gruppe bestehend aus Eisen (Fe), Magnesium (Mg), Aluminium (Al), Zink (Zn), Calcium (Ca), Mangan (Mn), Titan (Ti), Zirconium (Zr), Edelstahl, Gold (Au), Platinum (Pt), Iridium (Ir), Niobium (Nb), Silber (Ag), Tantalum (Tl), anderen Gefäßverträglichen Metallen und Kombinationen davon.

10. Medizinische Vorrichtung nach Anspruch 1, wobei der Wirkstoff ausgewählt ist aus der Gruppe bestehend aus Paclitaxel, Docetaxel, Estradiol, Stickstoffmonoxiddonatoren, Superoxid-Dismutasen, 4-Amino-2,2,6,6-tetramethylpiperidin-1-oxyl (4-Amino-TEMPO), Tacrolimus, Dexamethason, Rapamycin, 40-0-(2-Hydroxy)ethyl-rapamycin (Everolimus), 40-0-(3-Hydroxy)propyl-rapamycin, 40-042-(2-Hydroxy)ethoxy]ethyl-rapamycin, und 40-O-Tetrazol-rapamycin, 40-Epi-(N1-tetrazoyl)-rapamycin (ABT-578), Clobetasol, Pimecrolimus, Imatinibmesylat, Midostaurin, und Kombinationen davon.

11. Medizinische Vorrichtung nach Anspruch 1, die ein Stent ist.

12. Medizinische Vorrichtung nach Anspruch 1, wobei die mikroporöse Beschichtung eine keramische oder glasartige Beschichtung ist und eine anorganische Matrix umfasst, die ausgewählt ist aus der Gruppe bestehend aus Hydroxyapatit, Dahllit, Brushit, Octacalciumphosphat, Tricalciumfosfat, Calciumsulfat, Biogläsern, Carbiden, Tungstencabid, Kohlenstoff und Kombinationen davon.

13. Medizinische Vorrichtung nach Anspruch 1, wobei die mikroporöse keramische, metallische oder glasartige Beschichtung eine Verteilung der Mikroporengröße aufweist.

14. Medizinische Vorrichtung nach Anspruch 1, wobei den Adsorptions- oder Chemisorptionsnidus ausgewählt aus der Gruppe bestehend aus aktivem Kohlenstoff und Calciumcarbonat ist.

15. Medizinische Vorrichtung nach Anspruch 1, wobei der Adsorptions- oder Chemisorptionsnidus ausgewählt aus der Gruppe bestehend aus Fulleren und Mangan ist.

16. Medizinische Vorrichtung nach Anspruch 1 weiterhin umfassend eine oder mehrere Schicht(en) von Beschichtung eines Polymers, wobei die Schicht(en) von Polymerbeschichtung und die mikroporöse keramische, metallische oder glasartige Beschichtung eine schichtweise Konstruktion umfassen.

17. Medizinische Vorrichtung nach Anspruch 1, wobei die keramische oder glasartige Beschichtung eine anorganische Matrix umfasst, die ausgewählt ist aus der Gruppe bestehend aus Carbiden, Tungstencarbid, Kohlenstoff, Bioglass 45S5, Bioglass 45S5F, Bioglass 45S5.4F, Bioglass 40S5B5, Bioglass 52S4.6, Bioglass 55S4.3, A-W Glaskeramik, MB Glaskeramik, Zusammensetzungen aus SiO₂/NaO/CaO/P₂O₂, und Kombinationen davon.

18. Medizinische Vorrichtung nach Anspruch 1, wobei die keramische oder glasartige Beschichtung ein metallisches Material umfasst, das ausgewählt ist aus der Gruppe bestehend aus Zink (Zn), Mangan (Mn), Edelstahl, Iridium (Ir) und Kombinationen davon.

19. Verfahren zur Herstellung einer medizinischen Vorrichtung umfassend:
die Bildung einer mikroporösen, keramischen, metallischen oder glasartigen Beschichtung umfassend Makroporen und/oder Mikroporen,
wobei die Poren einen Adsorptions- oder Chemisorptionnidus umfassen, der ausgewählt ist aus der Gruppe bestehend aus Fullerenen, aktivem Kohlenstoff, Zeolit, Aliminiumsilicat, Calciumcarbonat, Chromium (Cr), Silica, Aluminiumoxid, Titandioxid, Gold (Au), Mangan (Mn) und Kombinationen davon
und
die Ladung eines Wirkstoffes in die Poren.

20. Medizinische Vorrichtung nach Anspruch 19, wobei die Beschichtung eine metallische Beschichtung ist und aus einem biologisch abbaubaren Metall ist, das hauptsächlich aus Zink, Magnesium oder Legierungen daraus besteht.

21. Verfahren nach Anspruch 19, wobei die Ladung des Wirkstoffes in die Poren ein Ionenaustauschverfahren umfasst.

## Revendications

1. Dispositif médical comprenant un revêtement microporeux céramique, métallique ou vitreux,
comprenant le revêtement des micropores chargés avec un agent bioactif et fournissant un profil de libération contrôlée de l'agent;
dans lequel les pores incluent un noyau d'absorption ou de chimisorption choisi dans le groupe constitué des fullerènes, du charbon activé, de la zéolite, du silicate d'aluminium, du carbonate de calcium, du chrome (Cr), de la silice, de l'alumine, du dioxyde de titane, de l'or (Au), du manganèse (Mn), et des combinaisons de ceux-ci.

2. Dispositif médical de la revendication 1, dans lequel le revêtement est un revêtement céramique ou vitreux et comprend une matrice inorganique choisie dans le groupe constitué de l'hydroxyapatite, de la dahlite, de la brushite, du phosphate d'octacalcium, du phosphate de tricalcium, du sulfate de calcium, de l'alumine, des verres biologiques, des carbures, du carbure de tungstène, du carbone, ou des combinaisons de ceux-ci.

3. Le dispositif médical de la revendication 1, dans lequel le revêtement est un revêtement métallique et c'est un métal biodégradable comprenant principalement de zinc, magnésium ou des alliages de ceux-ci.

4. Dispositif médical de la revendication 1, dans lequel le profil de libération est contrôlé moyennant un facteur choisi parmi la distribution de taille des micropores, le gradient de taille des micropores, l'épaisseur du revêtement, la tortuosité d'un réseau de micropores dans le revêtement, le facteur de rugosité de surface des micropores, le potentiel d'adsorption ou de chimisorption de l'agent sur la surface dans les micropores, une couche supérieure, ou une combinaison de ceux-ci.

5. Dispositif médical de la revendication 1, dans lequel le revêtement céramique, métallique ou vitreux a une fraction de volume de pores allant de 0,01 à 0,5.

6. Dispositif médical de la revendication 1, dans lequel le revêtement céramique, métallique ou vitreux comprend des macropores et des micropores avec un rapport de macropores à micropores allant de 0,01 à 0,99.

7. Dispositif médical de la revendication 1 comprenant en outre une couche supérieure de polymère sur le revêtement céramique, métallique ou vitreux.

8. Dispositif médical de la revendication 1, dans lequel l'agent bioactif est contenu dans une matrice ou microcapsule de polymère en particules.

9. Dispositif médical de la revendication 1, dans lequel le revêtement céramique, métallique ou vitreux comprend en outre un matériau métallique choisi parmi le groupe constitué du fer (Fe), du magnésium (Mg), de l'aluminium (Al), du zinc (Zn), du calcium (Ca), du manganèse (Mn), du titane (Ti), du zirconium (Zr), de l'acier inoxydable, de l'or (Au), du platinum (Pt), de l'iridium (Ir), du niobium (Nb), de l'argent (Ag), du tantalum (Tl), des autres matériaux vasculairement compatibles et des combinaisons de ceux-ci.

10. Dispositif médical de la revendication 1, dans lequel l'agent bioactif est choisi dans le groupe constitué du paclitaxel, du docétaxel, de l'estradiol, des donneurs d'oxyde nitrique, des superoxyde dismutases, du 4-amino-2,2,6,6-tétraméthypipéridine-1-oxyle (4-amino-TEMPO), du tacrolimus, de la déxaméthasone, de la rapamycine, de la 40-0-(2-hydroxy)éthyl-rapamycine (évérolimus), de la 40-0-(3-hydroxy)propyl-rapamycine, de la 40-042-(2-hydroxy)éthoxy]-éthyl-rapamycine, et de la 40-O-tétrazole-rapamycine, de la 40-epi-(N1-tétrazoyl)-rapamycine (ABT-578), du clobétasol, du pimécrolimus, du mésylate d'imatinib, de la midostaurine et des combinaisons de ceux-ci.

11. Dispositif médical de la revendication 1, qui est une endoprothèse vasculaire.

12. Dispositif médical de la revendication 1, dans lequel le revêtement microporeux est un revêtement céramique ou vitreux comprenant une matrice inorganique choisie dans le groupe constitué de l'hydroxyapatite, de la dahlite, de la brushite, du phosphate d'octacalcium, du phosphate de tricalcium, du sulfate de calcium, des verres biologiques, des carbures, du carbure de tungstène, du carbone, ou des combinaisons de ceux-ci.

13. Dispositif médical de la revendication 1, dans lequel le revêtement microporeux céramique, métallique ou vitreux a un gradient de taille des micropores.

14. Dispositif médical de la revendication 1, dans lequel le noyau d'absorption ou de chimisorption est choisi dans le groupe constitué du charbon activé et du carbonate de calcium.

15. Dispositif médical de la revendication 1, dans lequel le noyau d'absorption ou de chimisorption est choisi dans le groupe constitué du fullerène et du manganèse.

16. Dispositif médical de la revendication 1, comprenant en outre une ou plusieurs couche(s) de revêtement d'un polymère dans lequel le(s) couche(s) du revêtement de polymère et le revêtement microporeux céramique, métallique ou vitreux comprennent une construction couche par couche.

17. Dispositif médical de la revendication 1, dans lequel le revêtement céramique ou vitreux comprend une matrice inorganique choisie dans le groupe constitué des carbures, du carbure de tungstène, du carbone, du Bioglass 45S5, du Bioglass 45S5F, du Bioglass 45S5.4F, du Bioglass 40S5B5, du Bioglass 52S4.6, du Bioglass 55S4.3, du verre céramique A-W, du verre céramique MB, des compositions de SiO₂/NaO/CaO/P₂O₂ et des combinaisons de ceux-ci.

18. Dispositif médical de la revendication 1, dans lequel le revêtement céramique ou vitreux comprend un matériau métallique choisi dans le groupe constitué du zinc (Zn), de manganèse (Mn), de l'acier inoxydable, de l'iridium (Ir), et des combinaisons de ceux-ci.

19. Procédé de formation d'un dispositif médical comprenant:
la formation d'un revêtement microporeux céramique, métallique ou vitreux comprenant des macropores et/ou des micropores,
dans lequel les pores incluent un noyau d'absorption ou de chimisorption choisi dans le groupe constitué des fullerènes, du charbon activé, de la zéolite, du silicate d'aluminium, du carbonate de calcium, du chrome (Cr), de la silice, de l'alumine, du dioxyde de titane, de l'or (Au), du manganèse (Mn), et des combinaisons de ceux-ci
et
charger an agent bioactif dans les pores.

20. Le dispositif médical de la revendication 19, dans lequel le revêtement est un revêtement métallique et c'est d' un métal biodégradable comprenant principalement de zinc, magnésium ou des alliages de ceux-ci.

21. Procédé de la revendication 19, dans lequel l'étape de charge comprend charger l'agent bioactif dans les pores moyennant un procédé d'échange d'ions.
